# EUROPEAN PATENT APPLICATION

(11) **EP 1 110 967 A1**
(43) Date of publication of application: **27.06.2001**
(21) Application number: 99125485.5
(22) Date of filing: 21.12.1999
(51) Int. Cl.: C07H 21/00, C12Q 1/68

(54) **Compound comprising a biomolecule moiety and an organo-silane moiety**

(71) Applicant: LION Bioscience AG, 69120 Heidelberg (DE); VBC Genomics GmbH, 1091 Wien (AT)
(72) Inventor: Huber, Martin, Mag., 1030 Wien (AT); Hiller, Reinhard, Mag., 1120 Wien (AT); Schmidt, Wolfgang, Dr., 1030 Wien (AT); Müller, Manfred, Dr., 3400 Klosterneuburg (AT)

(57) **Abstract**

The present invention concerns a compound comprising a biomolecule moiety and an organo-silane moiety, as well as a process for the synthesis thereof. The invention also concerns a support comprising the biomolecule moiety with the organo-silane moiety, wherein the biomolecule moiety is attached to the support through the organo-silane moiety. The invention also concerns a process for a nucleic acid synthesis reaction making use of the biomolecule moiety with the organo-silane moiety as well as uses of the novel compound. The invention in addition concerns a kit comprising the compound comprising a biomolecule moiety and an organo-silane moiety.

SEQUENZPROTOKOLL

<110> Lion Bioscience AG
      VBC Genomics GmbH

<120> Compound Comprising a Biomolecule Moiety and an
      Organo-silane Moiety

<130> L-0008-00-EP-99

<140> EP
<141> 1999-12-21

<160> 2

<170> PatentIn Ver. 2.1

<210> 1
<211> 30
<212> DNA
<213> Künstliche Sequenz

<220>
<221> misc_structure
<222> (1)
<223> NH2-C6-

<220>
<223> Beschreibung der künstlichen Sequenz:
      künstliche

<400> 1
tttttttttt aagcgcgcaa ttaaccctca                                  30


<210> 2
<211> 20
<212> DNA
<213> Künstliche Sequenz

<220>
<223> Beschreibung der künstlichen Sequenz:
      künstliche

<400> 2
taaaacgacg gccagtgagc                                             20

## Description

### Background of the Invention

Genetic analysis often involves analysis of the nucleic acid sequence, structure or composition of a given organism or sample. Frequently, such analysis incorporates the step of, or requires nucleic acid amplification. One of the well known methods for nucleic acid amplification is the "PCR", or polymerase chain reaction method also disclosed in US 4,683,195 and US 4,683,202. Here, a nucleic acid sample serves as a template for a polymerase dependant *in-vitro* replication starting from two separate primers. Polymerases are enzymes capable of synthesizing RNA or DNA making use of RNA or DNA as a template. Often times the analysis is performed on RNA (ribonucleic acid), here amplification additionally requires an enzymatic reverse transcription into DNA (deoxyribonucleic acid), but equally often on DNA. PCR is becoming powerful tool in diagnostics. PCR kits are becoming available for the detection and analysis of various pathogenic organisms as well e.g. mutant alleles of human genes.

PCR is mostly performed *in-vitro, i.e.* in a tube whereby the components are mostly supplied in liquid format. Alternatively, one or more of the components, these usually being, a polymerase, a buffer, a template, two or more oligonucleotides, may be bound to some form of a solid-phase.

One very common problem with the PCR being performed in a standard non solid-phase format is the limitation with respect to the number of primer pairs that may be used simultaneously in one reaction. In contrast a solid-phase set-up would theoretically enable the use of tens to thousands of primer pairs.

More recently, it has been proposed to use one primer bound to a solid-support together with a free primer in solution in order to simultaneously amplify and bind a PCR product onto a surface (Oroskar, A. A., Rasmussen, S. E., et al., Detection of immobilized amplicons by ELISA-like techniques, Clinical Chemistry 42:1547 (1997)). The drawback of this approach is that the actual multiplexing , *i.e.* the use of a multitude of primer pairs in one reaction is not really facilitated as the free primers in solution may still spuriously anneal.

A solid-phase method is disclosed in US 5,641,658 or WO 96/04404 here, the oligonucleotides used in the reaction are bound to a support. Such a set-up would have a number of advantages over the standard PCR process, *e.g.* it would be easy to combine a multitude of primer pairs in one reaction without the drawbacks of false amplification products from false primer pairings. Thus, multiplexing would be facilitated.

A problem with such an approach is the efficiency of the reaction, thus also the product yield during amplification is poor and consequently the reliability of the entire process. Often these problems are associated with the fact that the reaction conditions *i.e.* the availability of the primer in the reaction are not optimal. One may envision that the 3' OH ends of the oligonucleotides are not available due to the fact that parts of the oligonucleotide are bound to the support. Such immobilized single-stranded DNAs which have conventionally been used are prepared by binding a single-stranded DNA at the terminal molecule or a suitable functional group introduced into the molecule. However, such conventional method has a drawback in that it is impossible to bind the single-stranded DNA to the carrier only at its terminal molecule by the use of the conventional method because an existing amino or hydroxy group, or other functional group artificially introduced, on the nucleotide molecules other than terminal also participates in the binding with the carrier. The immobilized DNA obtained with conventional methods is one in which the DNA molecules are bonded to the support at various sites of the strand. As will be recognized by those skilled in the art, such a molecule is poorly suited for providing sufficient experimental results in any aspects.

A further problem is that the molecules bound to the support are sterically hindered by the support itself from taking part in *e.g.* enzymatic reactions. EP0787205 discloses the use of linker between the oligonucleotide and the solid-support. However, the primary problem is not addressed here. The primers on a solid-support are not freely available in the reaction. Thus primers remain a limiting factor.

For efficient amplification to occur it would be desired to provide equimolar amount of each primer of a given pair of primers. Due to the fact that primers are bound to a support where an unequal distribution and binding of primers will occur stochastically it is impossible to achieve equimolar amounts of primer in the reaction.

Often reactive groups are used to bind the Primers to the support. Such reactive groups have been *e.g.* amino groups. It is known in the art that such groups are very unstable, consequently when a given primer pair is arrayed on a support wherein the terminal group of the primers are e.g. amino groups it is to be expected, based on the instability of the groups, that after arraying the primers in each pair will not be present equimolar manner.

One method for the covalent attachment of oligonucleotides on glass supports is to treat the glass with an aminosilane and couple a 5' amino-modified oligonucleotide via covalent bond formation using 1,4-Phenylene-diisothiocyanate (DITC) (Guo et al. 1994 NAR 22:5456-5465). This procedure has two major technical drawbacks. As outlined above, the terminal primary amino group is unstable and therefore also not useful for long-term storage which is one pre-requisite in DNA chip technology. One should mention here that glass slides are often chosen as solid support in molecular DNA analysis and often referred to as DNA chips. Second, the generation of an aminoreactive glass support requires complete inactivation of unreacted groups after spotting in order to avoid a high background which in fact makes multiple solid-phase approaches entirely impossible.

It was therefore an object of the present invention to provide for a process, means and substances that lead to a high efficiency in *in-vitro* solid-phase nucleic acid synthesis reactions, thus also to a higher product yield and consequently a higher reliability of such reactions.

It was further an object of the present invention to provide for a process, means and substances to be used in *in-vitro* solid-phase nucleic acid synthesis reactions that are suited for providing good experimental results in many aspects such as, equimolarity of oligonucleotide amount, reduction of background signal, reduction of falsely synthesized products, availability of oligonucleotides in the reaction, high adsorption capacity of bound oligonucleotide to solid.

It was a further object within the concept of the invention to provide for novel molecules capable of solving the above problems. Thus it was *e.g.* an object of the present invention to provide for molecules that are readily available in a DNA synthesis reaction and not sterically hindered by *e.g.* a solid-support or the like.

It was a also an object within the concept of the invention to provide for processes for making the molecules according to the invention.

It was a further object within the concept of the invention to provide for a kit comprising one or more of the molecules according to the invention.

Equally it was an object of the present invention to provide for a kit comprising compounds necessary for performing the process according to the invention.

Further objects of the invention are apparent to the skilled person from the specification.

### Summary of the Invention

The objects of the present invention were accomplished by providing for a compound with novel characteristics which may be used in solid phase enzymatic reactions, a processes for making this compound, a processes for in vitro nucleic acid synthesis for use with and without the novel molecules as well as kits containing a compound according to the invention for use in processes according to the invention as well as other processes.

The object of the present invention was accomplished by providing for a compound comprising a biomolecule moiety and an organo-silane moiety as represented in formula 1 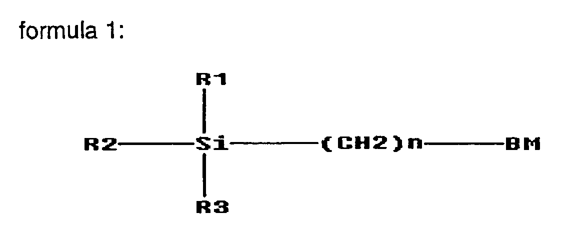 wherein, R₁, R2, and R3 are identical or different alkoxy groups and "BM" represents the biomolecule moiety or a derivative thereof and wherein, n is an integer from 0 to 18. This novel compound has surprisingly shown unexpected results when compared to similar molecules previously used in solid-phase enzymatic reactions with respect to but not limited to the following effects, its adsorption capacity to a solid-support, it's availability in enzymatic reactions, thus its contribution to the efficiency of e.g. solid-phase nucleic acid synthesis reactions.

The alkoxy groups R₁, R₂, and R₃ may, e.g. by methoxy, ethoxy or the like. Within the scope of the invention are organo-silane moieties comprising mixtures of different alkoxy groups. For example, R₁ may be a methoxy, R2 an ethoxy and R3 a methoxy. The skilled artisan is credited with the ability to discern alternative combinations which shall be within the scope of the invention.

Herein, solid phase reactions and solid-support reactions are used with equal meaning and shall be understood as such reactions in which one or more compounds is attached to a solid matter of any given shape or chemical structure.

Herein, a biomolecule is to be understood as any molecule which shows enzymatic activity, which acts as a probe in molecular analysis or which is the target of an enzymatic activity.

In a preferred embodiment biomolecules are nucleic acids of natural or synthetic origin. Nucleic acids may be DNA or RNA. The DNA or RNA may be single-, double-or triple-stranded. If the nucleic acid is of synthetic origin it may be enzymatically, e.g. by PCR or chemically synthesized. In the event the nucleic acid is of synthetic origin and chemically synthesized this may be performed with the "phosphoramidite methodology", see e.g. U.S. Pat No. 4,415,732; McBride L. and Caruthers, M.

*Tetrahedron Letters* 24:245-248 (1983); and Sinha N. et al. Nucleic Acids Res. 12:4539-4557 (1984), which are all incorporated herein by reference. The group of nucleic acids within the scope of the invention shall encompass but is not limited to the nucleic acid forms cited above. In particular derivatives of the above are within the scope of the invention, such as PNA-DNA hybrid oligonucleotides (see Finn, P. J. et al., N.A.R. 24, 3357-3363 (1996), Koch, T. et al., Tetrahedron Letters 36, 6933-6936 (1995), Stetsenko, D. A. et al., Tetrahedron letters 37, 3571-3574 (1996), Bergmann, F et al., Tetrahedron Letters 36 6823-6826 (1995) and Will, D. W. et al., Tetrahedron 51, 12069-12082 (1995)).

Also, one or more amino acids, peptides as well as proteins may be represented by the term biomolecules "BM" within the scope of the invention.

The compound according to the invention comprises an organo-silane as well as a biomolecule. It is obvious to one skilled in the art, that these two moieties may but must not be connected through one or more methylene groups. The compound according to the invention may thus also comprise the organo-silane group which is directly coupled to the methylene group.

In a preferred embodiment of the compound according to the invention however, the organo-silane group is bound to between 1 and 18 methylene groups which are bound to the biomolecule.

In a preferred embodiment of the compound according to the invention the biomolecule within the compound is a nucleic acid, more particularly an oligonucleotide moiety or an analog. Oligonucleotides within the scope of the invention are ordinarily single stranded and comprise between 1 and about 100 nucleotides. Ordinarily these are linked by a standard phosphodiester bond however, they may be linked also by peptide bonds such as in the case of PNAs (Egholm, M., Buchardt, O., Nielsen, P.E. and Berg, R.H. "Peptide nucleic acids (PNA): Oligonucleotide analogues with an achiral peptide backbone" J. Am. Chem. Soc. 114 (1992) 1895-1897; Nielsen, et al. "Peptide nucleic acids (PNA): Oligonucleotide analogues with a polyamide backbone" Antisense Research and Applications (1992) 363-372). The oligonucleotides or oligonucleotide analogs may however, also be of branched type structure such as Y shaped or T shaped (Horn T, Urdea MS "Forks and combs and DNA: the synthesis of branched oligodeoxyribonucleotides" Nucleic Acids Res (1989) 17: 6959-67; Horn T, Chang CA, Urdea MS "Chemical synthesis and characterization of branched oligodeoxyribonucleotides (bDNA) for use as signal amolifiers in nucleic acid quantification assays" Nucleic Acids Res (1997) 25: 4842-4849).

In a preferred embodiment of the invention the oligonucleotides or oligonucleotide analogs are capable of binding a nucleic acid molecule through hybridization and comprise between 5 and 30 nucleotides.

The inventors have found that synthesis of the compound according to the invention is facilitated if the compound according to the invention further comprises a linking moiety R4 interpost between the organo-silane moiety and the biomolecule moiety as represented by formula 2 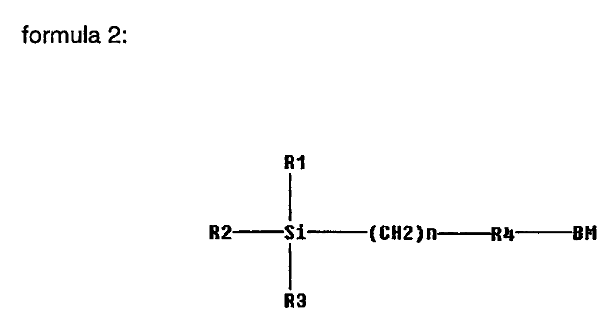 wherein, R₁, R2, and R3 are identical or different alkoxy groups and BM represents the biomolecule moiety or a derivative thereof, n is an integer from 0 to 18 and wherein, R4 represents the linking moiety.

The compound according to the invention is preferentially synthesized with the aid of homo- or hetero-bifunctional groups. These groups are used to specifically connect a methyl group or alternatively the organo-silane with the biomolecule. These groups result in the linking moiety R₄ after reacting. Thus, such a linking moiety within the scope of the invention is to be understood as any moiety stemming from a homo- or hetero-bifunctional group after having reacted with an organo-silane and a biomolecule.

The inventors have found that the organo-silane reacts both with other compounds according to the invention well, which may be desirable as outlined below as well as with various solid supports well if R₁, R₂, and R₃ are each methoxy groups. Thus in a preferred embodiment of the invention R₁, R₂, and R₃ are each methoxy groups.

The biomolecule is preferentially coupled via a bifunctional linking moiety R₄ to the organo-silane. It has been found that there are particularly suited bifunctional linking reagents for accomplishing this. Such bifunctional linking reagents may be selected from the group comprising arylenediisothiocyanate, alkylenediisothiocyanate, bis-N-hydroxy-succinimidylesters, hexamethylenediisocyanat and N-(γ. maleimidobutyryloxy)succinimide ester.

Hence, once the bifunctional linking reagent has reacted with the adapter molecule "AM" or alternatively directly with the biomolecule "BM" at its first reactive group and the organo-silane at its second reactive group a linking moiety R₄ is present in the compound according to the invention. In a preferred embodiment of the invention R₄ is selected from the group comprising arylene(bisthiourea) and alkylene(bisthiourea).

In a preferred embodiment of the compound according to the invention the linking molecule R₄ is phenylenebisthiourea.

The inventors have found that the synthesis of the compound according to the invention is greatly facilitated if the biomolecule comprises a reactive group on a separate moiety which enables the binding to R₄ or alternatively to the organo-silane.

In the case of nucleic acids and oligonucleotides in particular such an adapter moiety may be characterized by formula 5, 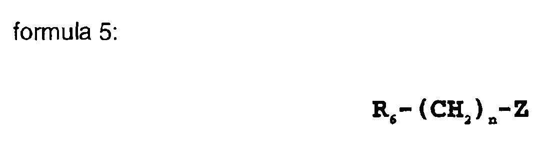 wherein R₆ is selected from the group comprising cyanoethylphosphoramidites, wherein Z is selected from the group comprising -NH₂, -SH and, n is an integer from 0 to 18. Thus in a preferred embodiment of the invention the compound further comprises an adapter moiety "AM" interpost between the linking moiety R₄ and the molecule BM as represented by the formula 3, 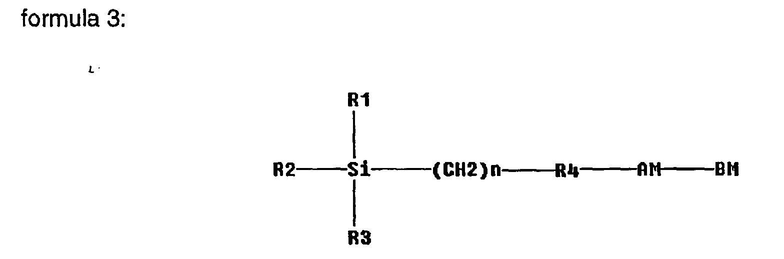 wherein, R₁, R₂, and R₃ are identical or different alkoxy groups, BM represents the biomolecule moiety or a derivative thereof, n is an integer from 0 to 18, R₄ represents the linking moiety and wherein "AM" is the adapter moiety.

In a preferred embodiment of the invention the adapter moiety "AM" is chosen from the group comprising -(CH₂)ₙ and -[(CH₂)₂O]ₙ wherein n is an integer from 0 to 18.

It can be shown that particularly good results are obtained with oligonucleotides in enzymatic reactions if the organo-silane is coupled terminally to the nucleic acid. Thus, in a preferred embodiment of the invention the biomolecule moiety BM is linked terminally via its 3' or 5' end to the linking moiety R4 or alternatively to the adapter moiety AM whereas in case the biomolecule BM is linked to the linking moiety R₄ directly there is no adapter moiety AM present and whereas in case the biomolecule BM is linked to the adapter moiety AM is linked to the linking moiety R4 or directly to the organo-silane. Here, terminally shall mean the end of a linear nucleic acid. Natural nucleic acids have a 3'end and a 5' designating which of the carbon atoms of the sugar moiety is free and terminal.

The inventors have found that a very particular compound according to the invention is easy to synthesize and shows excellent results in solid-phase experiments. This compound is represented by formula 4

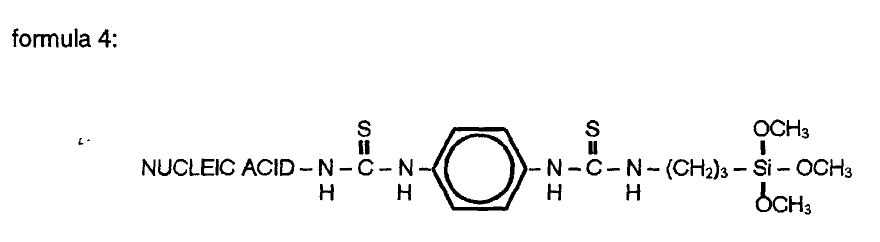

The process for the synthesis of the compound according to the invention may at first glance seem fairly straight forward that is in hind sight. However, the prior art to the knowledge of the inventors discloses no examples of the coupling of a biomolecule BM to an organo-silane.

The invention also covers a process for the synthesis of a compound as disclosed above. In this process, 1) an organo-silane is reacted with a biomolecule BM wherein, the organo-silane is represented by formula 5: 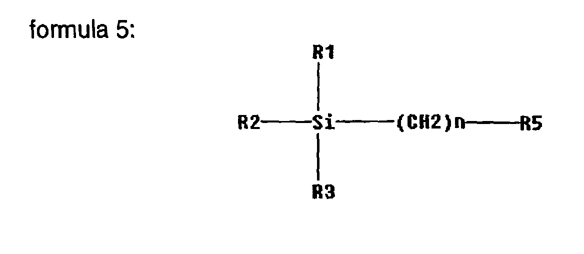 wherein, R₁, R₂, and R₃ represent identical or different alkoxy groups, wherein R₅ is selected from the group comprising -NH₂ (-amino), -SH (-sulfhydryl), -NCO (-cyanato), -NHS ester (hdroxysuccinimidylester, -acrylate) and n is an integer from ( to 18.

The inventors have found that the above process is facilitated if prior to the reactior between the organo-silane and the biomolecule, BM is reacted with an adapter molecule AM and subsequently reacted with the organo-silane. One reason for this simply that e.g. if the biomolecule is a nucleic acid in particular an oligonucleotide such an adapter molecule is easily coupled during on-line synthesis.

While one skilled in the art will come up with various adapter molecules and thus th invention shall not be limited by the following example, the inventors have found the adapter molecule AM as represented by formula 5 is preferred.

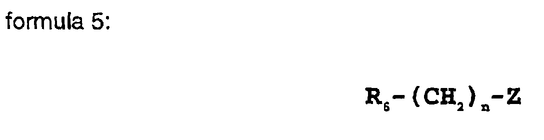

Here R₆ is selected from the group comprising cyanoethylphosphoramidites, Z is selected from the group comprising -NH₂, -SH, -PO₄, -COOH, -I and, n is an integer from 0 to 18.

### Claim: 14

In a preferred embodiment in the process according to the invention the biomolecule BM is reacted with a linking molecule R₄ and subsequently reacted with the organo-silane or alternatively, the organo-silane is reacted with the linking molecule R₄ and subsequently reacted with the biomolecule BM wherein, the linking molecule R₄ is a bifunctional reagent. Although the process according to the invention does not require such a linking molecule R₄ the inventors find this to be advantageous.

As outlined above it is preferred that the linking moiety is a bifunctional reagent, *i.e* a coupling reagent with two reactive groups. In a preferred embodiment of the process according to the invention the linking molecule R₄ is selected from the group comprising arylenediisothiocyanate, alkylenediisothiocyanate, bis-N-hydroxy-succinimidylesters, hexamethylenediisocyanate and N-(γ-maleimidobutyryloxy)succinimide ester. It is evident that these are preferred examples and one skilled in the art may find other possible bifunctional reagents which are equally within the scope of the invention.

In a preferred embodiment the linking molecule R₄ is 1,4-phenylene diisothiocyanate.

The inventors have coupled the compound according to the invention to glass supports (see example 1 and 2) and performed solid-support nucleic acid synthesis reactions. Also the amount of bound nucleic acid was measured before and after washing (see example 4). Astonishingly when these figures are evaluated it is found that when binding an oligonucleotide carrying an amino modification to a pretreated glass slide as this has been done up the conception and reduction to practice of the present invention on average only 17.3 % whereas 91% remained bound to the surface when the oligonucleotides or oligonucleotide analogs according to the invention were used. In addition, the ratio calculated from the aminosilane treated slides between bound oligo and background (control) is 5.4 to 1 for the oligonucleotides or oligonucleotide analogs according to the invention the ratio is 151 to-1.

This has drastic implications for the use of the compound according to the invention. Only to name a few of the advantages this incurs e.g. the background reduction will make it possible to perform solid-phase quantification experiments such as from nucleic acids much more precisely, the bottom detection limit of analytes such as nucleic acids will drop, thus more precise results will be obtainable in various fields where the compound according to the invention finds applications.

The compound according to the invention is preferentially used in solid-phase reactions here the compound may be bound to substances chosen from the group comprising nitrocellulose, nylon, controled-pore glass beads (CPG), polystyrene, activated dextran, modified polystyrene, styrene-acrylnitril-copolymers, polycarbonate, cellulose, polyamide and glass.

The inventors have astonishingly found that a support comprising a compound according to the invention exhibits an adsorptions capacity of at least 1 pmol of compound according to the invention per mm², often 10 pol/mm² and mostly about 80 pmol/mm². These high figures are not achievable when applying prior-art technology.

In a preferred embodiment the compound is used with glass although it may also be used in combination with any other solid-support. Such glass may be a glass slide, as used *e.g.* for microscopy, glass vessels or containers, glass fibers, glass beads or other Si comprising glass entities.

The compound according to the invention may be spotted onto, pipetted onto, sprayed onto or otherwise brought onto such a glass support. Possible methods are, application by means of a needle, capillary, dispenser and piezo pipette is preferable, *e.g.* an apparatus similar to the kind known for ink jet printers.

The compound according to the invention may be used in various ways some of which shall be mentioned here. The compound is particularly suited for nucleic acid hybridization or synthesis reactions. Here, the compound may be bourid to a solid support such as glass. The compound represents one or more nucleic acid probes to which a target, *i.e.* the sample is bound. Such hybridization experiments are disclosed in WO 95/00530.

The compound according to the invention may be used to distinguish single base mismatches. In U.S. Pat. 5,700,638 such experiments are described in Example 2.

US Pat. 5,552,270 also describes such an approach. Here, the compound according to the invention comprises an oligonucleotide of defined sequence. An array is generated comprising numerous different sequences each suited to test a defined sequence.

The compound according to the invention may be used to analyze the expression of genes. Here, oligonucleotides or nucleic acid fragments, e.g. PCR products represent the BM according to the invention which are attached to a suited solid-support to form an array of various probes, labeled RNA or pre-amplified RNA is hybridized to the array and the positive hybridizations scored as expressed genes. Such methods are described in GB Pat. 2318791.

The compound according to the invention may be used to map genomes of organisms. Here, oligonucleotides or nucleic acid fragments, e.g. PCR products represent the BM according to the invention which are attached to a suited solid-support to form an array of various probes, labeled genomic fragments are sequentially hybridized in numerous steps in such a way that the relationship between individual fragments becomes apparent. Such an approach is disclosed in U.S. Pat. 5,219,726.

The compound according to the invention may comprise enzymatic functions as BM or nucleic acids as BM. In each case this facilitates solid-support enzymatic reactions. In a preferred embodiment of the invention the compound is used for nucleic acid synthesis reactions. Here, the BM of the compound is preferentially a nucleic acid with one or more free 3' OH groups which may be template dependently extended by a polymerase.

In a preferred embodiment the BM are primers which are attached to a solid glass support and thus the compound according the invention may be used to perform PCR on glass.

In a preferred embodiment the BM are primers which are attached to a solid-support and a reverse transcription reactions of RNA into DNA is performed.

The invention shall also cover a process for a nucleic acid synthesis reaction of one or more selected regions of one or more target nucleic acids comprising the steps of a) combining the sample containing the target region with at least on nucleotide triphosphate, a thermally stable polymerase, a buffer and at least one primer wherein the primer is the BM of the compound according to the invention, b) exposing the reaction mixture to at least one temperature cycle including at least a high temperature denaturation phase and a lower temperature extension phase, and thereby producing at least a partially amplified product.

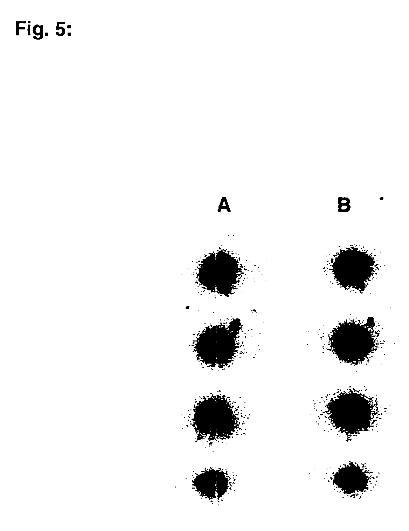

The inventors have found that the compound according to the invention gives unexpectedly good results when used on solid-phase in *in-vitro* DNA synthesis reactions (see also example 1 and Fig. 5).

Thus in a preferred embodiment of the above process at least one compound according to the invention is bound to a solid-support.

This process for a nucleic acid synthesis reaction makes use of oligonucleotide, *i.e.* a primer, as the BM moiety of the compound according to the invention. Such primers comprise preferably between 3 and 100 nucleotides more preferably between 10 and 50 nucleotide most preferably between 12 and 25 nucleotides. It is essential in this context that the nucleic acid is of sufficient length to bind the desired target nucleic acid molecule with sufficient stringency. PNA-DNA hybrid oligonucleotides (see Finn, P. J. et al., N.A.R. 24, 3357-3363 (1996), Koch, T. et al., Tetrahedron Letters 36, 6933-6936 (1995), Stetsenko, D. A. et al., Tetrahedron letters 37, 3571-3574 (1996), Bergmann, F et al., Tetrahedron Letters 36 6823-6826 (1995) and Will, D. W. et al., Tetrahedron 51, 12069-12082 (1995)) are also considered as primers for the process of the present invention.

As a thermally stable polymerase, a DNA polymerase may be selected from the group comprising Taq DNA polymerase, Tth DNA polymerase or Klentaq (Taq DNA polymerase (-exo5'-3'), Korolev et al., (1995) Proc. Natl. Acad. Sci. USA 92, 9246-9268. The use of Taq DNA polymerase in the method of the present invention is especially preferred.

Alternatively as a thermally stable polymerase, a DNA polymerase which has a decreased discrimination against the four ddNTPs with respect to wild-type Taq DNA polymerase in the buffer or under the conditions used for thermal cycling is preferred. More preferably, a DNA polymerase Taq polymerase carrying a "Tabor-Richardson" mutation or a functional derivative thereof which also lacks 5'-3' exonuclease activity such as, for example, AmplitaqFS^{TM} (Taq DNA polymerase (-exo5'-3')(F667Y), Tabor and Richardson (1995), loc. cit.), Taquenase^{TM} (Taq DNA polymerase Δ235(-exo5'-3')(F667Y), Tabor and Richardson (1995), loc. cit.) and Thermosequenase^{TM} (Taq DNA polymerase (-exo5'-3')(F667Y), Tabor and Richardson (1995), loc. cit.) as well as mixtures thereof or other DNA polymerases and mixtures thereof which are thermally stable can be used in the process of the present invention. The use of Thermo Sequenase^{TM} or any other DNA polymerase having a high ability to incorperate ddNTPs in the method of the present invention is especially preferred.

Alternatively as a thermally stable polymerase, a DNA polymerase which has a decreased discrimination against labeled nucleotide may be used.

The present invention, i.e. the process also provides for the use of two or more polymerases in the process or additional enzymes such as amplification enhancing reagents such as thermostable pyrophosphatase or enzymes which enhance the processivity of the polymerase such as PCNA (proliferating cell nuclear antigen) homologues. Enzyme mixtures may be equally applied.

The number of thermal cycles may range from about 1 to about 50 depending on the amount of template DNA and its purity. Generally, the inventors have found that very surprisingly extremely short cycles give good results. As the availability of the compound according to the invention is high in the process according to the invention the cycle period may be short, thus disadvantageous denaturing of proteins, e.g. the polymerase when in contact with glass occurs at a lower rate and the reaction may run efficiently without loss of function of enzyme.

Routinely, cycling consists of (i) a denaturing cycle, (ii) an annealing cycle and (iii) an extension cycle. Alternatively, only two cycles may be applied, (i) a denaturing cycle and (ii) an annealing and extension cycle.

Preferably the denaturing cycle is performed at between 100°C and 85°C, more preferably at between 98°C and 90°C, most preferably at between 96°C and 92°C. Preferably the annealing cycle is performed at between 80°C and 45°C, more preferably at between 70°C and 50°C, most preferably at between 60°C and 55°C.

Preferably the extension cycle is performed at between 80°C and 50°C, more preferably at between 75°C and 60°C, most preferably at between 73°C and 68°C.

Preferably the denaturing cycle is performed for 3 minutes, more preferably for 30 seconds, most preferably for under 10 seconds.

Preferably the annealing cycle is performed for 3 minutes, more preferably for 30 seconds, most preferably for under 10 seconds.

Preferably the extension cycle is performed for 3 minutes, more preferably for 30 seconds, most preferably for under 10 seconds, however the extension time vary depending on the length of the template, in particular the extension time may be raised if the template length increases.

Buffers components which can be used can include, but are not limited to, Tris-HCI at a pH of about 7.0 to 10 and concentration of about 2 to 60 mM, ammonium sulfate at a concentration of about 10-20 mM, preferably 15 mM, MgCl₂ at a concentration of about 1 to 10 mM, and optionally, about 0.05 mM mercaptoethanol, about 0.28% Tween® 20 and/or about 0.02% Nonidet® 40.

Nucleotide triphosphates are preferably deoxynucleotides and can be selected from, but are not limited to, dGTP, dATP, dTTP and dCTP. In addition, derivatives of deoxynucleotides, which are defined as those deoxynucleotides which are capable of being incorperated by a thermally stable DNA polymerase into nascent DNA molecules synthesized in the thermal cycling reaction, can also be used according to the invention. Such derivatives include, but are not limited to thionucleotides, 7-deaza-2'-dGTP, 7-deaza-2'-dATP as well as deoxyinosine triphosphate which may also be used as a replacement deoxynucleotide for dATP, dGTP, dTTP or dCTP. The above mentioned deoxynucleotides and derivatives thereof are preferably used at a concentration of about 50 µM to about 4 mM.

Preferable the nucleotides are mixes of all four and at 200 µM per nucleotide.

In a preferred embodiment one or more of the nucleotides incorporated are labeled. For example, single and differential labels may consist of the group comprising enzymes such as β-galactosidase, alkaline phosphatase and peroxidase, enzyme substrates, coenzymes, dyes, chromophores, fluorescent, chemiluminescent and bioluminescent labels such as FITC, Cy5, Cy5.5, Cy7, Texas-Red and IRD40(Chen et al. (1993), J. Chromatog. A 652: 355-360 and Kambara et al. (1992), Electrophoresis 13: 542-546), ligands or haptens such as biotin, and radioactive isotopes such as ³H, ³⁵S, ³²P ¹²⁵l and ¹⁴C.

In one embodiment of the method of the invention, the nucleic acid molecule to be amplified can be present in the form of total genomic DNA, which is preferably in an uncloned or unpurified form. Preferably, the genomic DNA has a length greater than or equal to 2 kb. Generally all forms of template may be used, e.g. purified nucleic acids, *i.e.* nucleic acids where one fraction may be enriched or not, one example being plasmid DNA the other purified genomic DNA. The process may be suited for use with complex mixtures of DNA such being purified but not substantially fractionated genomic DNA or non-complex mixtures such being purified and substantially fractionated DNA e.g. plasmid DNA.

In a further preferred embodiment of the method of the invention, the nucleic acid molecule to be amplified can be present in the form of RNA. One polymerase or a mixture of two polymerases maybe utilized: a first DNA polymerase for example, Taq polymerase, and a second DNA polymerase with the capability to reverse transcribe RNA into DNA preferably Taq DNA polymerase (Jones et al., Nucl. Acids Res. 17: 8387-8388 (1989)) or Tth DNA polymerase (Myers et al., Biochemistry 30: 7666-7672 (1991 )).

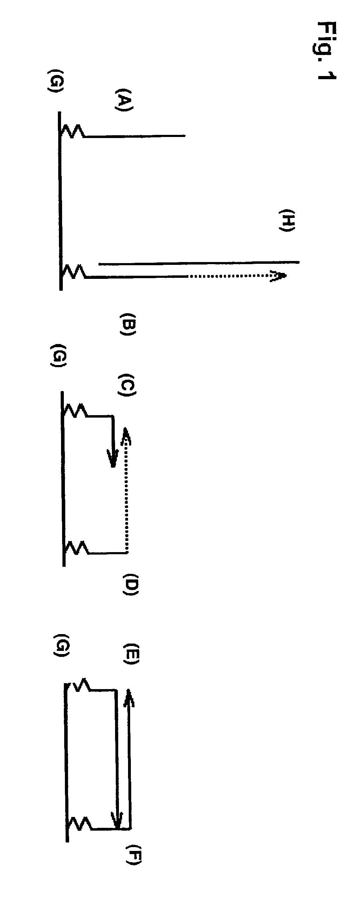

In one embodiment of the invention the process is performed with Y-shaped oligonucleotides as BM. Here the terminal parts, *i.e.* the Y ends are free 3'OH ends and each arm is either a sense or an antisense oligonucleotide (see Fig. 1, 2, and 3).

The invention also covers a kit for use in molecular biology or chemistry comprising at least the compound according to the invention. The kit may also comprise other reagents or enzymes such as buffers, nucleotides or the like.

While the foregoing has been set forth in detail, the Examples are presented for elucidation, and not limitation. Modifications and improvements on the compound and or process according to the invention disclosed above which are within the purview and abilities of those in the art are included within the scope of the claims.

### Example 1: Coupling a compound according to the invention to a glass support.

Oligonucleotides were synthesized using standard cyanoethyl posphoramidite chemistry on an ABI 392 DNA/RNA Synthesizer. Primary amino groups with an hexyl carbon atom spacer were introduced using the N-MMT-C6-aminomodifier (Cruachem Cat. No. 22-8401-17). Oligonucleotides were synthesized using the trityl-on mode and purified via HPLC (BioCad, PE Biosystems) using Oligo R3 reversed phase chromatography media (PE Biosystems Cat. No.1-1339-06) and an on-column detritylation standard protocol.

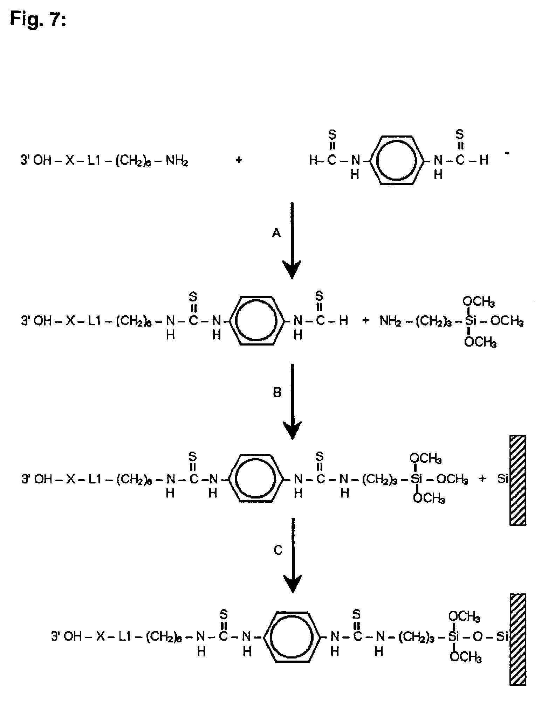

The purified oligonucleotide or single branched amplification molecule containing the 5' hexylamino modification (100 pmoles) was incubated in 100 µl of a 100 mM 1,4-phenylene diisothiocyanate (DITC) containing solution of pyridine : dimethylformamide (ratio 1: 9) for 2 hours at ambient temperature (See also Fig. 7 reaction A).

After standard precipitation with ethanol/sodium acetate the oligonucleotide was dissolved in 100 µl of a 1, 3-aminopropyltrimethoxysilane solution in 95 % acetone/sodium hydroxide (100mM) and incubated for 2 hours at ambient temperature (See also Fig. 7 reaction B). After standard precipitation the compound according to the invention bearing the glass-reactive silane group was re-dissolved in 95 % aqueous acetone and stored at 4 °C. Attachment to various supports, such as glass was accomplished by spotting (See also Fig. 7 reaction C).

### Example 2: Comparing the compound according to the invention with prior art technology

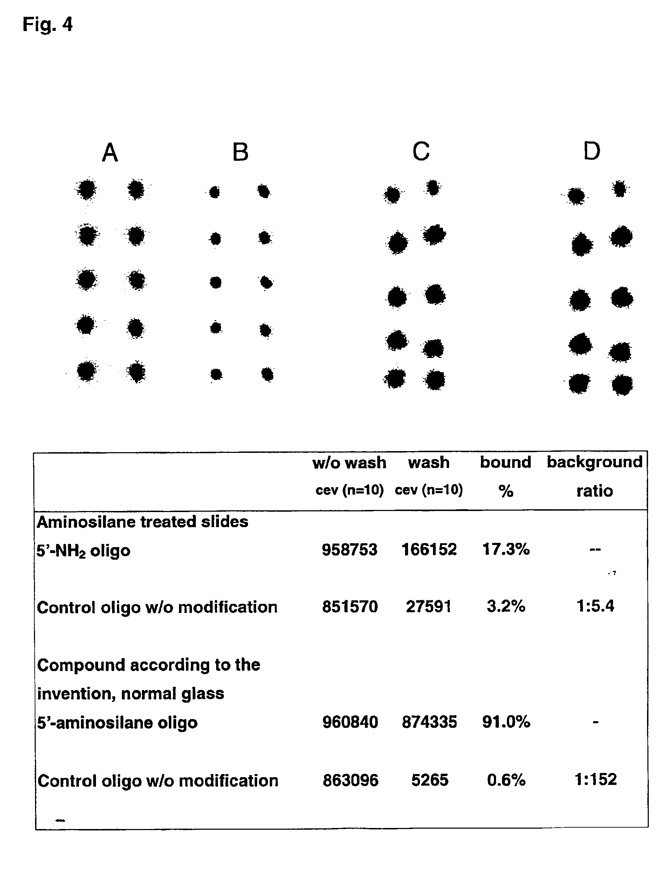

In order to asses the efficiency of binding of oligonucleotides or oligonucleotide analogs carrying a terminal silane group a comparison between silane treated slides and oligonucleotides or oligonucleotide analogs carrying a terminal silane group according to the invention was performed. Oligonucleotides were radioactively 3'-end labeled using terminal transferase and ³²P-α-ddATP according to a standard protocol and purified via acrylamide gel electrophoresis. The purified labeled oligonucleotides (1 µl, 10.000 cpm) were spotted onto the respective glass slides using a micropipette. Oligonucleotides carrying a terminal silane group were spotted onto normal glass slides, whereas oligonucleotides with terminal aminohexyl modification were spotted onto silane treated slides. As a control also oligonucleotides without any terminal modification were spotted onto both types of slides (see Table in Fig. 4).

All slides were scanned directly after spotting using a Phosphor Imager (Molecular Dynamics Model 400).

Silane treated slides were incubated for 2 h at ambient temperature in a humid chamber and subsequently rinsed with 1 % ammonia, washed with 1 % SDS for 10 min, washed with water and finally dried and scanned again using the Phosphor Imager.

Normal untreated glass slides were also washed with 1 % SDS for 10 min, washed with water and finally dried and scanned again using the Phosphor Imager.

Figure four shows that whereas only 17.3% of the oligonucleotides or oligonucleotide analogs invested remained on the solid-phase when the silane group had been established on the solid-phase surface, more than four times more, *i.e.* 91% remained bound to the surface when the oligonucleotides or oligonucleotide analogs according to the invention were used. In addition, the ratio calculated from the aminosilane treated slides between bound oligo and background (control) is 5.4 to 1 for the oligonucleotides or oligonucleotide analogs according to the invention the ratio is 151 to 1 (see Table in Fig. 4).

### Example 3: Assessment of binding capacity of the compound according to the invention

In order to assess the binding capacity of the compound according to the invention, *i.e* an organo-silane modified oligonucleotide, to a normal entirely untreated glass slide, 1 nmol of an amino-modified oligonucleotide (30-mer) was spiked with 1 fmol of gel-purified 3'-end labeled oligonucleotide (labeled as described in example 2; 10.000 cpm) and reacted with a 100 mM solution of 1 -[3"-(trimethoxysilyl)propyl]-1'-(4"-isocyanatophenyl) thiourea in 1,4-dioxan for 10 min at ambient temperature. 1 µl of the oligonucleotide solution was then spotted onto normal untreated glass slides using a micropipette incubated for 10 min at ambient temperature, scanned using a Phosphor Imager (Molecular Dynamics Model 400), washed with 1 % SDS for 10 min, rinsed with water and finally dried and scanned again. Quantification was performed using the ImageQuant Software (Molecular Dynamics). The result is shown in Fig. 5, 98.7 % (n= 4) of the silane-treated oligonucleotides were covalently bound to the glass support on spots with a diameter of approximately 4 mm. This corresponds to a binding capacity of approximately 80 pmol / mm².

### Example 4 (solid-phase amplification):

Specific oligonucleotides for the amplification of a 209 bp fragment of the pBluescript KS+ polylinker sequence were synthesized using standard cyanoethyl posphoramidite chemistry on an ABI 392 DNA/RNA Synthesizer. Oligonucleotides were synthesized using the trityl-on mode and purified via HPLC (BioCad, PE Biosystems) using Oligo R3 reversed phase chromatography media (PE Biosystems Cat. No.1-1339-06) and an on-column detritylation standard protocol. Primary amino groups with an hexyl carbon atom spacer were introduced using the N-MMT-C6-aminomodifier (Cruachem Cat. No. 22-8401-17).

In order to introduce the organo-silane modification to the aminolink containing oligonucleotide ("BSfor") the synthesis cartridge was removed from the Synthesizer after deprotection of the terminal MMT group with 2 % (v/v) trifluoracetic acid (TFA). 2 ml of a solution containing 0.5 % (w/v) 1,4-phenylene diisothiocyanate in dimethylformamide : pyridine 1:9 were applied slowly to the cartridge and pushed forth and back using two syringes. After a 30 min incubation at ambient temperature the cartridge was washed 3 times with 2ml dimethylformamide : pyridine 1:9, washed once with 10 ml acetonitril and dried using an argon flush. In a next step 2ml of a solution containing 1% (v/v) 1,3-aminopropyltrimethoxysilane in dimethylformamide : pyridine 1:9 were applied to the cartridge followed by an incubation at ambient temperature for 2 hours. After washing with dimethylformamide : pyridine 1:9 the silane-modified oligonucleotides were cleaved from the support and deprotected by incubating in ammonium hydroxide at 55 °C for 6 hours, evaporated to dryness by vacuum centrifugation and dissolved in water at a concentration of 1 nM. Attachment to glass-slides was accomplished by spotting using a GMS 417 Arrayer (Genetic Microsystems) at 10 hits per dot (corresponds to approximately 5 pmoles oligonucleotide applied to the glass surface in a spot of 150µm diameter).

Sequence of the oligonucleotides:
SEQ ID NO. 1:
"BSfor": 5' NH2-C6-TTT TTT TTT TAA GCG CGC AAT TAA CCC TCA 3'
SEQ ID NO. 2:
"BSrev": 5' TAA AAC GAC GGC CAG TGA GC 3'

Amplification reactions were performed in a 5 µl volume containing 50 mM Tris-HCI at pH 8.5, 30 mM KCI, 3 mM magnesium chloride, 0.5 µg bovine serum albumine (the inventors found it to be very important to use high amounts of BSA), 500 µM each dGTP, dATP and dTTP, 300 µM dCTP, 20 µM Cy5-dCTP (Amersham Pharmacia Biotech, Cat. No. PA 55021), 10 pmoles oligonucleotide "BSrev", 5 units Taq DNA Polymerase and 3 fmole of template DNA (pBlueScript (KS +) plasmid DNA). The reaction chamber was formed by overlaying the reaction droplet on the slide with a glass cover slip and sealing with Canada Balsam (Sigma Cat. No. C 1795). The slide was then attached to a modified frame (originally designed for holding glass capillaries) which fits to the reaction chamber of the thermal-cycler device (Idaho Technology, Thermo-Cycler 1605). Reactions were cycled with the following profile: one initial cycle at 94°C for 10 seconds, thirty cycles at 94 °C for 2 seconds , at 60°C for 2 seconds and at 74 °C for 15 seconds, and one final cycle for 30 seconds at 74°C.

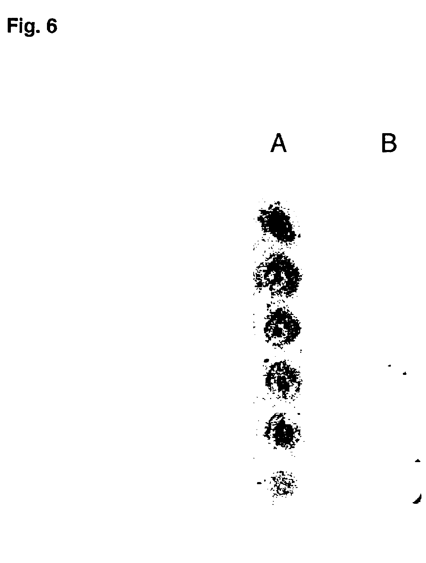

After the incubation in the thermal-cycler slides were removed and the cover slips lifted off. The slides were transferred to a micro-array washing device (Telechem International, Arraylt Microarray Wash Station) and rinsed with 0.2 % SDS for 15 minutes, cleaned in water for 15 minutes and dried at ambient temperature. Scanning of the fluorescent amplification products was accomplished in a fluorescent scanning device (Genetic Microsystems, Array Scanner GMS 418). The results are shown in Fig. 6.

### Figure Captions:

### Fig.1:

Fig. 1 shows a schematic representation of a solid-phase in-vitro nucleic acid synthesis reaction. The figure shows a compound according to the invention wherein, the biomolecule moiety as an arrow and an organo-silane moiety as zig-zag line, both coupled via the organo-silane moiety to a solid support (G). Here, an *in-vitro* nucleic acid synthesis reaction is schematically shown. A target molecule (H) anneals to the biomolucule (B) where it is used as a template and the biomolecule is extended by a polymerase. Thereafter, the extended biomolecule may serve as a template for a further biomolecule (C) which must be in sufficiently close vicinity. The biomolecule (C) now uses the template (D) and is extended to the full-length product (E). As schematically shown here, efficient synthesis relies on equimolarity and close vicinity of two or more different biomolecules required in one reaction.

### Fig. 2:

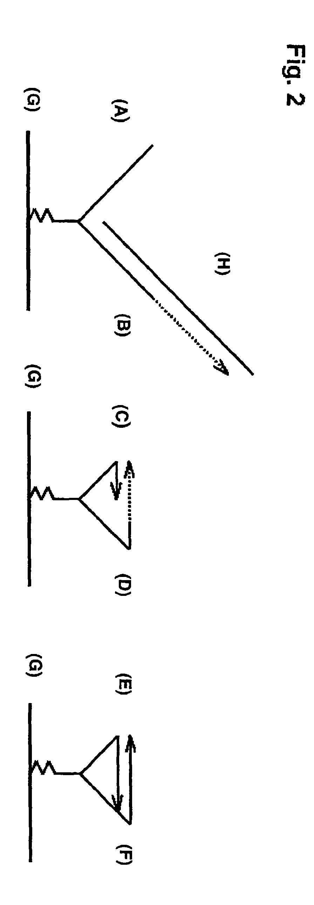

Fig. 2 shows a schematic representation of a solid-phase *in-vitro* nucleic acid synthesis reaction. The figure shows a preferred compound according to the invention wherein, the biomolecule moiety is a branched compound comprising two free 3'-OH groups and an organo-silane moiety as zig-zag line, both coupled via the organo-silane moiety to a solid support (G). Here, an *in-vitro* nucleic acid synthesis reaction is schematically shown. A target molecule (H) anneals to the biomolucule (B) where it is used as a template and the biomolecule is extended by a polymerase. Thereafter, the extended biomolecule may serve as a template for a further biomolecule (C) which is by definition in sufficiently close vicinity. The biomolecule (C) now uses the template (D) and is extended to the full-length product (E). As schematically shown here, efficient synthesis relies on equimolarity and close vicinity of two or more different biomolecules required in one reaction.

### Fig. 3:

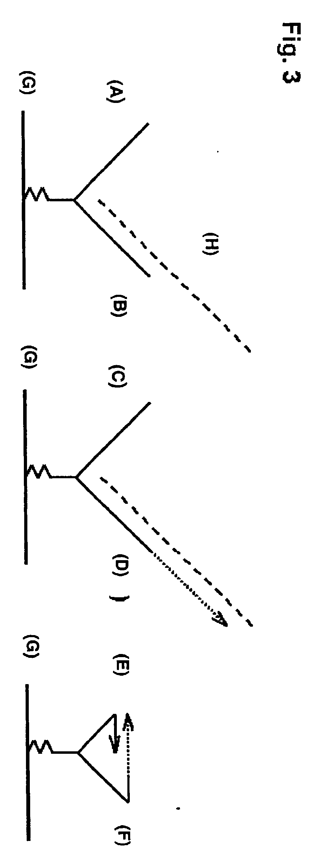

Fig. 3 shows a schematic representation of a solid-phase *in-vitro* nucleic acid synthesis reaction starting from mRNA including the step of prior reverse transcription coupled to an amplification step. The figure shows a preferred compound according to the invention wherein, the biomolecule moiety is a branched compound comprising two free 3'-OH groups and an organo-silane moiety as zig-zag line, both coupled via the organo-silane moiety to a solid support (G). Here (A to F), an *in-vitro* nucleic acid reverse transcription with coupled amplification reaction is schematically shown. A target mRNA molecule (H) anneals to the biomolecule (B) where it is used as a template and the biomolecule is extended by a polymerase with reverse transcriptase activity. Thereafter, the extended biomolecule may serve as a template for a further biomolecule (C) which is by definition in sufficiently close vicinity. The biomolecule (C) now uses the template (D) and is extend to the full-length product (E). As schematically shown here, efficient synthesis relies on equimolarity and close vicinity of two or more different biomolecules required in one reaction. The compound according to the invention takes place in reverse transcription as well as subsequent amplification.

### Fig. 4:

Fig. 4 (results to example 2) shows a comparison between binding amino-modified oligonucleotides to silane-treated glass (panels A, B) and binding oligonucleotides carrying a terminal silane group to normal glass (panels C, D) according to the invention. The 3' ³²P-labeled oligonucleotides (10.000 cpm) were spotted onto the respective glass slides and scanned (panels A, C). As a control also unmodified oligonucleotides were spotted onto both types of slides (not shown, see Table) to determine unspecific background binding. After extensive washing and rinsing steps the slides were dried and scanned again (panels B, D). Herein, cev (counts equivalent value) were determined by quantification of the Phosphor Imager gel scans using the ImageQuant software.

### Fig. 5:

Fig. 5 (results to example 3) shows an experiment for the determination of the binding capacity of an organo-silane modified oligonucleotide to normal untreated glass. 1 nmol of an amino-modified oligonucleotide was spiked with 1 fmol of gel-purified 3'-end labeled oligonucleotide (10.000 cpm) and derivatized with an organo-silane according to the invention, spotted four times onto the slides and scanned (panel A). The slides were then washed, dried and scanned again (panel B). 98,7 % of the silane-treated oligonucleotides were covalently bound to the glass support.

### Fig. 6:

Fig. 6 (results to example 4) shows the on-glass amplification of a specific target with an immobilized primer using the organo-silane derivatization according to the invention. The second primer was contained in the reaction solution. The figure shows the fluorescent image of the amplification reaction (panel A) and control reaction (panel B) without template DNA, 6 spots each. The incorporation of fluorescent dye results from the specific amplification of a 209 bp fragment of pBlueScript KS+ DNA. The amplicon is immobilized on the glass support via the covalently bound amino-silane treated oligonucleotide.

### Fig. 7:

Fig. 7 shows schematically the reactions leading to the derivatization of an oligonucleotide with an organo-silane moiety according to a preferred embodiment of the invention. An oligonucleotide containing a 5' hexylamino modification is first reacted with 1,4-phenylene diisothiocyanate (reaction A) and further reacted with 1, 3-aminopropyltrimethoxy-silane (reaction B). The compound bearing the glass-reactive silane group can then be attached to *e.g.* glass supports (reaction C).

## Claims

1. Compound comprising a biomolecule moiety and an organo-silane moiety as represented in formula 1 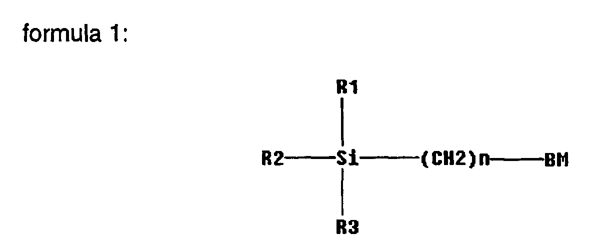 wherein, R₁, R₂, and R₃ are identical or different alkoxy groups and BM represents the biomolecule moiety or a derivative thereof and wherein, n is an integer from 0 to 18.

2. Compound according to claim 1 wherein,
the biomolecule BM comprises a nucleic acid, more particularly an oligonucleotide moiety.

3. Compound according to claim 1 or 2
further comprising a linking moiety R₄ interposted between the organo-silane moiety and the biomolecule moiety as represented by formula 2 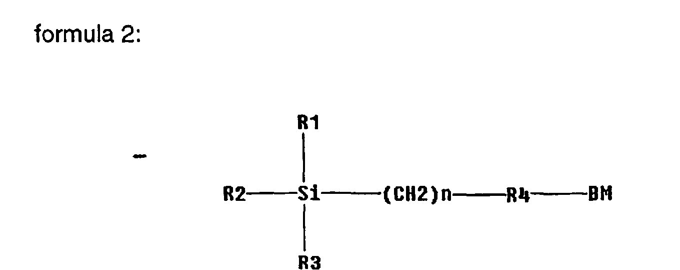 wherein, R₁, R₂, and R₃ are identical or different alkoxy groups and BM represents the biomolecule moiety or a derivative thereof, n is an integer from 0 to 18 and wherein, R₄ represents the linking moiety.

4. Compound according to claim 1 wherein,
R₁, R₂, and R₃ are each methoxy groups.

5. Compound according to claim 4 wherein,
R₄ is selected from the group comprising arylene(bisthiourea) and alkylene(bisthiourea).

6. Compound according to claim 5 wherein,
R₄ is phenylenebisthiourea.

7. Compound according to any of claims 1-6 wherein,
the compound further comprises an adapter moiety "AM" interposted between the linking moiety R₄ and the molecule BM as represented by the formula 3, 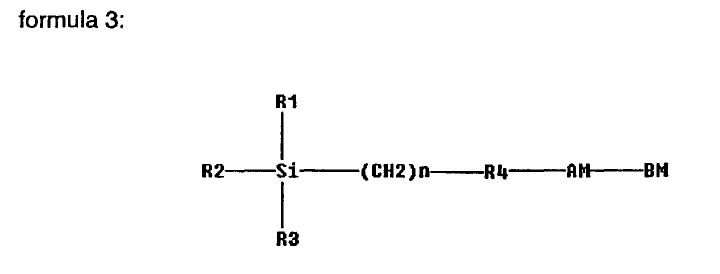 wherein, R₁, R₂, and R₃ are identical or different alkoxy groups, BM represents the biomolecule moiety or a derivative thereof, n is an integer from 0 to 18, R₄ represents the linking moiety and wherein "AM" is the adapter moiety.

8. Compound according to claim 7 wherein,
the adapter moiety is selected from the group comprising -(CH₂)n, -[(CH₂)₂O]n wherein n is an integer from 0 to 18.

9. Compound according to any of claims 2-8 wherein,
the biomolecule moiety BM is linked terminally via its 3' or 5'end to the linking moiety R4 or alternatively to the adapter moiety AM whereas in case the biomolecule BM is linked to the linking moiety R₄ directly there is no adapter moiety AM present and whereas in case the biomolecule BM is linked to the adapter moiety AM is linked to the linking moiety R₄ or directly to the organo-silane.

10. Compound according to claim 2 to 9 wherein,
the compound is represented by formula 4

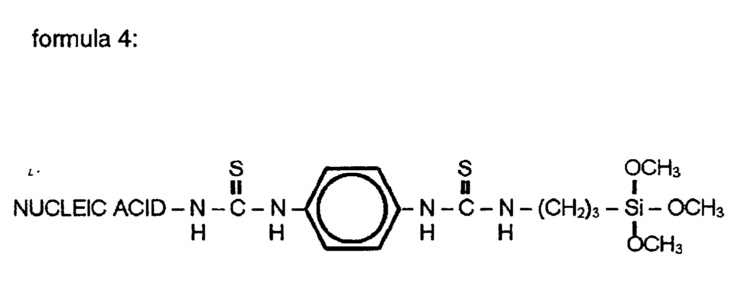

11. Process for the synthesis of a compound according to claims 1 to 10, characterized in that,
1) an organo-silane is reacted with a biomolecule BM wherein, the organo-silane is represented by formula 5: 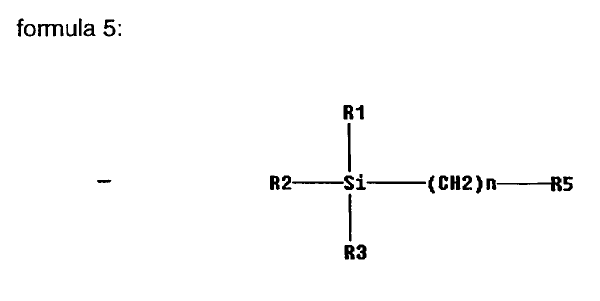 wherein, R₁, R₂, and R₃ represent identical or different alkoxy groups,
wherein R₅ is selected from the group comprising -NH₂, -SH, -COOH, -PO₄, -1, N-hydroxy-succinimidylester and n is an integer from 0 to 18.

12. Process according to claim 11,
characterized in that the biomolecule BM is reacted with an adapter molecule AM and subsequently reacted with the organo-silane.

13. Process according to claim 12,
characterized in that the adapter molecule AM is represented by formula 5, 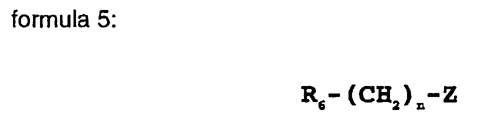 wherein R₆ is selected from the group wherein R₆ is selected from the group comprising cyanoethylphosphoramidites,
wherein Z is selected from the group comprising -NH₂, -SH, -PO₄, -COOH, -I and,
n is an integer from 0 to 18.

14. Process according to any of the claims 11 to 13,
characterized in that the biomolecule BM is reacted with a linking molecule R₄ and subsequently reacted with the organo-silane or alternatively,
organo-silane is reacted with the linking molecule R₄ and subsequently reacted with the biomolecule BM wherein,
the linking molecule R₄ is a bifunctional reagent.

15. Process according to claim 14,
characterized in that the linking molecule R₄ is selected from the group comprising arylenediisothiocyanate, alkylenediisothiocyanate, bis-N-hydroxy-succinimidylesters, hexamethylenediisocyanate and N-(γ-maleimidobutyryloxy)succinimide ester.

16. Process according to claim 15,
characterized in that the linking molecule R₄ is is 1,4-phenylene diisothiocyanate.

17. Support comprising a compound according to any of the claims 1 to 10,
wherein the support exhibits an adsorptions capacity of at least 1 pmol/mm², preferably 10 pol/mm² and most preferably 80 pmol/mm².

18. Support comprising a compound according to any of the claims 1 to 10,
wherein the support is obtainable by a process according to any of the claims 11 to 16.

19. Support comprising a compound according to any of the claims 1 to 10,
wherein the support is chosen from the group comprising nitrocellulose, nylon, controled-pore glass beads (CPG), polystyrene, activated dextran, modified polystyrene, styrene-acrylnitril-copolymers, polycarbonate, cellulose, polyamide and glass.

20. Use of a biomolecule according to claims 1 to 10,
in nucleic acid synthesis reactions.

21. Use of a biomolecule according to claims 1 to 10,
in primer extension reactions.

22. Use of a biomolecule according to claims 1 to 10,
in reverse transcription reactions of RNA into DNA.

23. Use of a biomolecule according to claims 1 to 10,
in nucleic acid hybridization experiments.

24. Use of a biomolecule according to claims 1 to 10,
in nucleic acid hybridization experiments for identifying the sequence of a nucleic acid.

25. Use of a biomolecule according to claims 1 to 10,
in nucleic acid hybridization or amplification experiments for analyzing the expression pattern of genes.

26. Use of a biomolecule according to claims 19 to 24,
whereby the biomolecule is bound to a solid-support.

27. Process for a nucleic acid synthesis reaction of one or more selected
regions of one or more target nucleic acids comprising the steps of
a) combining the sample containing the target region with at least on nucleotide triphosphate, a polymerase, a buffer and at least one primer according to claim 1-10,
b) exposing the reaction mixture to at least one temperature cycle including at least a high temperature denaturation phase and a lower temperature extension phase, and thereby producing at least a partially amplified product.

28. Process according to claim 27,
characterized in that at least one compound according to claims 1 to 10 is bound to a solid-support.

29. Kit comprising the compound according to any of the claims 1 to 10.
